# EUROPEAN PATENT APPLICATION

(11) **EP 2 107 059 A1**
(43) Date of publication of application: **07.10.2009**
(21) Application number: 08153750.8
(22) Date of filing: 31.03.2008
(51) Int. Cl.: C07D 471/04, C07D 487/14

(54) **Conversion of tryptophan into ß-carboline derivatives**

(71) Applicant: LEK Pharmaceuticals d.d., 1526 Ljubljana (SI)
(72) Inventor: The designation of the inventor has not yet been filed
(74) Representative: Opresnik, Marko

(57) **Abstract**

The invention belongs in the field of organic chemistry and relates to a new shortened Pictet-Spengler type reaction for preparing isomerically pure β-carboline compounds useful for the synthesis of tadalafil.

## Description

### Field of the Invention

The present invention belongs to the field of organic chemistry and relates to a new shortened Pictet-Spengler type reaction for preparing isomerically pure β-carboline compounds.

More specifically the present invention relates to the reaction of the D-tryptophan with piperonal in a solvent in the presence of an inorganic acid to yield a cis-β-carboline compound which is further reacted with chloroacetyl chloride and methylamine to yield tadalafil.

### Background of the Invention

Tadalafil (Formula **1**), (6R,12aR)-2,3,6,7,12,12a-hexahydro-2-methyl-6-(3,4-methylenedioxyphenyl)-pyrazino[2',1':6,1]pyrido[3,4-b]indole-1,4-dione, is a pharmaceutically active substance from the group of selective inhibitors of phophodiesterase (PDE5) and is used in the treatment of erectile dysfunction. The molecule was first disclosed in WO 95/19978 in a wider group of potentially active molecules.

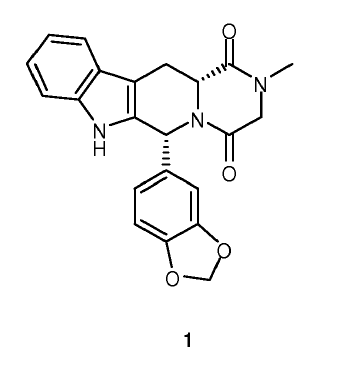

The first preparative approach was disclosed in the above mentioned patent application and is shown in Scheme 1:

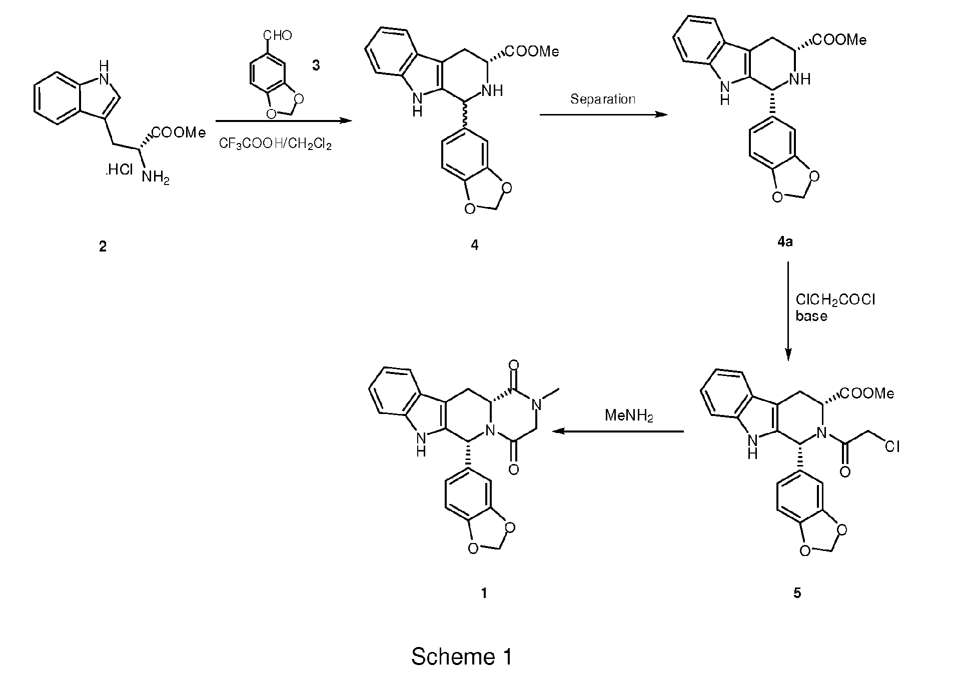

Briefly described, the process involves condensation of D-tryptophan methyl ester (**2**) with piperonal (**3**) to give a mixture of cis and trans isomers of β-carboline ester (**4**) which is separated by using column chromatography. The cis isomer (**4a**) is treated with chloroacetyl chloride to give the substituted carboline (**5**) which is finally cyclized with methylamine into tadalafil. The critical step in the synthetic approach is the so-called Pictet-Spengler reaction in the first step. Use of volatile and corrosive trifluoroacetic acid, separation of cis and trans β-carbolines and low yields are the main drawbacks for an effective industrial process.

Later literature descriptions tried to surpass the deficiencies of this approach. WO 04/011463 gives up the use of trifluoroacetic acid and uses solvents in which cis isomer (**4a**) precipitates during the Pictet-Spengler reaction. This causes spontaneous transformation of the trans isomer into cis isomer (**4a**) and considerably improves the yield. WO 05/068464 uses molecular sieves to improve the yield, WO 06/110893 uses less toxic solvents, WO 07/052283 uses high boiling point solvents. But all these approaches use D-tryptophan methyl ester (**2**) which is not a commonly used raw material. It is usually prepared from D-tryptophan (**6**) with thionyl chloride which is a user unfriendly reagent for the industry.

The first attempt to use D-tryptophan in the acid form (**6**) in the Pictet-Spengler reaction is described in WO 07/100387 (Scheme 2) but a mixture of cis and trans isomers of β-carboline acid (**7**) is obtained and isolated and then transformed into the pure cis isomer (**7a.HCl**) with a 40 hour long heating in an aqueous hydrochloric acid solution having a concentration of 2 equivalents related to tryptophan. Cis-β-carboline acid is then transformed with N-methylglycine esters and coupling reagents into crude tadalafil which is finally purified.

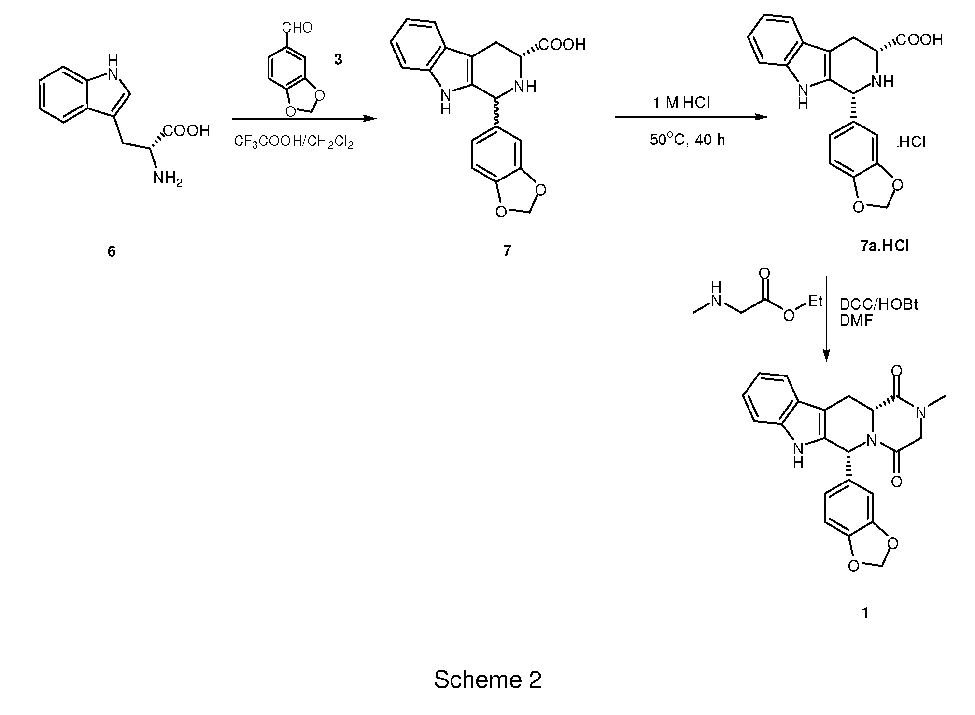

No reference has been identified to disclose a one pot reaction of an isomeric tryptophan with piperonal to obtain cis-β-carboline compounds.
Therefore there still exists a problem to shorten and simplify the Pictet-Spengler procedure in the synthesis of β-carbolines.

### Summary of the Invention

The first embodiment of the present invention provides a Pictet-Spengler process for preparing isomerically pure β-carboline compounds by the reaction of the suitable isomeric tryptophan with piperonal in a solvent in the presence of an inorganic acid.

Another embodiment of this invention provides a reaction of D-tryptophan and piperonal into cis-β-carboline compound in a solvent in the presence of an inorganic acid.

Another embodiment of this invention provides a reaction of L-tryptophan and piperonal into cis-β-carboline compound in a solvent in the presence of an inorganic acid.

Another embodiment of this invention provides a reaction of D-tryptophan and piperonal into cis-β-carboline carboxylic acid in an aprotic solvent in the presence of inorganic acids in one step without a separation of cis and trans isomer, or isomerization.

Another embodiment of this invention provides a transformation of cis-β-carboline carboxylic acid to obtain tadalafil.

Another embodiment of this invention provides a reaction of D-tryptophan and piperonal into cis-β-carboline esters in alcohols in the presence of inorganic acids in one step without a preparation and isolation of the tryptophan ester.

Another embodiment of this invention provides a transformation of cis-β-carboline ester with chloroacetyl chloride and methylamine to obtain tadalafil.

Another embodiment of this invention provides use of an inorganic acid in the coupling of tryptophan and piperonal.

Another embodiment of this invention provides a pharmaceutical composition comprising tadalafil whereby tadalafil is prepared from D-tryptophan.

Another embodiment of this invention provides use of tadalafil prepared according to one of the processes disclosed in this invention, for the preparation of a medicament for the treatment of erectile dysfunction or related disorders.

Another embodiment of this invention provides a pharmaceutical formulation comprising at least one pharmaceutically acceptable excipient and tadalafil prepared according to one of the processes disclosed in this invention.

Another embodiment of this invention provides use of tadalafil prepared according to one the processes disclosed in this invention for the preparation of the pharmaceutical formulation together with at least one pharmaceutically acceptable ingredient.

### Detailed Description of the Invention

The Pictet-Spengler reaction has been known as a condensation of β-amino alkyl substituted indoles with aldehydes into β-carbolines in the presence of a stronger inorganic or organic acid. In a general approach of the synthesis of tadalafil, D-tryptophan esters and piperonal have been used. If using D-tryptophan in the acid form instead of esters and performing the reaction in conventional media such as alcohols or chlorinated solvents, only complex mixtures are obtained which should be further converted into pure cis-β-carbolines. But we surprisingly found that if the Pictet-Spengler reaction between D-tryptophan in the acid form and piperonal is carried out in the mixture of at least one non-chlorinated aprotic solvent and at least one inorganic acid, only one isomer is obtained and there is no need for a further conversion from trans to cis isomer or the separation thereof (Scheme 3).

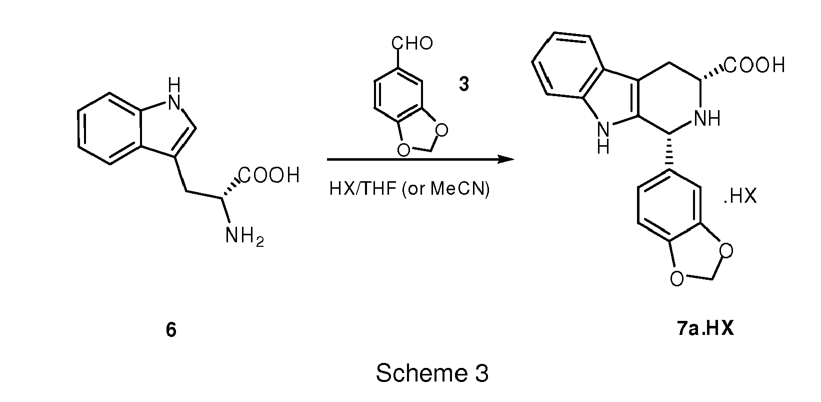

Such solvents are selected from the group of ethers, esters and nitriles, preferably from 1,4-dioxane, tetrahydrofuran, 2-methyl-tetrahydrofuran and acetonitrile.

The inorganic acid is selected from strong inorganic acids, preferably hydrochloric acid, hydrobromic acid, and sulphuric acid, more preferably hydrochloric acid.

Thus D-tryptophan is suspended in 5 to 100, preferably 8 to 30 ml per g of tryptophan of a non-chlorinated aprotic solvent, such as alkyl ethers, alkyl esters, 1,4-dioxane, tetrahydrofuran, 2-methyl-tetrahydrofuran and acetonitrile. Piperonal (3) is added, followed by the addition of 1 to 5 equivalents of hydrochloric acid, preferably 1.1 to 2 equivalents, more preferably 1.1 to 1.9 equivalents, even more preferably 1.1 to 1.3 equivalents. The mixture is heated to about 40 °C to reflux temperature, preferably to reflux temperature and stirred at this temperature for 16 hours to 10 days, preferably for 3 to 4 days. The resulting slurry is filtered to give the desired (**7a.HCl**) as a pure cis form (1 R,3R-β**-**carboline).

In one way of carrying out the present invention, the obtained acid (**7a.HCl**) can be transformed into tadalafil without converting to esters, following a known procedure using carbodiimide condensing methods.

In another conventional way, the acid (**7a**) is first converted to alkyl ester. Thus said acid is heated in a C₁-C₄ alcohol, preferably selected from methanol, ethanol, n-propanol; in the presence of an inorganic acid, selected from strong inorganic acids, preferably hydrochloric acid, hydrobromic acid, sulphuric acid. The carboxylic acid is dissolved in 10 to 100 ml per g of tryptophan, preferably in 20 to 50 ml per g of tryptophan of alcohol, followed by the addition of 1 to 10 equivalents, preferably 1.1 to 2 equivalents, more preferably 1.1 to 1.9 equivalents, even more preferably 1.1 to 1.3 equivalents of an inorganic acid. The mixture is heated to about 40 °C to reflux temperature, preferably to about 60 °C to reflux temperature, and the mixture is stirred at this temperature for 12 to 72 hours, preferably for 24 to 36 hours. Solvents are then evaporated under the reduced pressure to give an approximately 1:1 mixture of cis and trans isomers of (**8**).

The obtained mixture of cis and trans isomers of (**8**) is routinely converted into pure cis isomer (**8a**) as it is described elsewhere and the obtained product is further transformed to chloroacetyl derivative (**5**) by the reaction with chloroacetyl chloride in dichloromethane in the presence of triethylamine or another suitable base. This derivative is then reacted with methylamine in an alcohol in the presence of a suitable base to obtain tadalafil.

In another embodiment of the invention, there was an attempt to prepare the intermediate acid (7a.HX) by using a Pictet-Spengler condensation in alcoholic media using catalytic amounts of an inorganic acid. Usually catalytic amounts are sufficient, for instance in the condensation of D-tryptophan methyl ester hydrochloride (**2**) and piperonal, the hydrochloride moiety is sufficient to make the reaction feasible. If piperonal is reacted with D-tryptophan in the acid form (**6**) in methanol in the presence of catalytic amounts of an inorganic acid, only complex mixtures are obtained. But we surprisingly found that in excess of the inorganic acid, the reaction takes place in a clearer way and also involves esterification of the carboxylic group with a minimal residue of the unesterified carboxylic acid. The completeness of the esterification is also surprising in view of the general fact that the reaction between the acid and the alcohol is equilibrated as it is seen in the preparation of tryptophan esters wherein the esterification in acidic methanol is not complete and thionyl chloride must be used. The transformation is shown in Scheme 4:

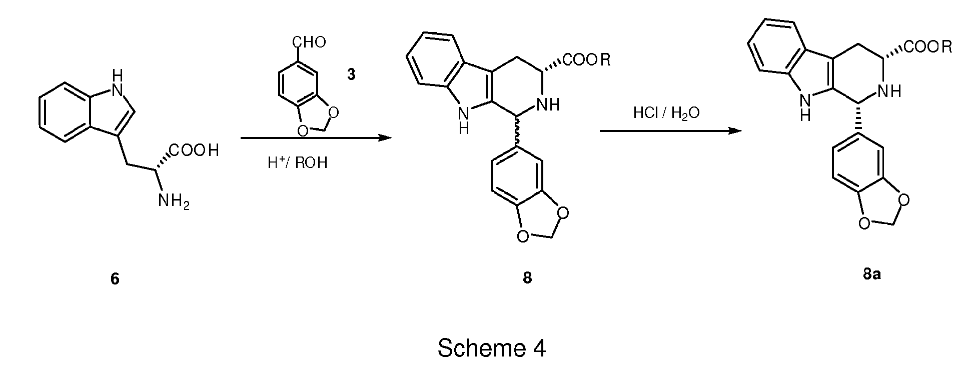

Thus D-tryptophan is suspended at room temperature in 5 to 50 ml per g of tryptophan, preferably 6 to 12 ml per g of tryptophan of a C₁-C₄-alcohol preferably selected from methanol, ethanol or *n*-propanol and piperonal is added. The mixture is then treated with an inorganic acid, selected from strong inorganic acids, preferably hydrochloric acid, hydrobromic acid, sulphuric acid, more preferably hydrochloric acid, either as concentrated aqueous solutions or as gaseous hydrogen chloride or hydrogen bromide. The amount of the acid used is between 1 and 5 equivalents, preferably between 1.2 and 2 equivalents. The solution is heated to about 40 °C to reflux temperature, preferably to 60 to 70 °C and stirred at this temperature for 12 hours to 6 days, preferably for 3 to 4 days. The solvent is then partially evaporated and the rest is dissolved in 10 to 50 ml per g of tryptophan, preferably in 20 to 30 ml per g of tryptophan of dichloromethane or ethyl acetate and washed with 10 to 30 ml per g of tryptophan of 1 N aqueous solution of NaOH. The organic phase is evaporated to give an approximately 1:1 mixture of cis and trans isomers of (**8**), in which R represents C₁-C₄ -alkyl, preferably methyl or ethyl.

This mixture of cis and trans isomers of (**8**) can then be routinely converted into the pure cis isomer (**8a**) and then further on to tadalafil as already described above.

Furthermore the Pictet-Spengler condensation and isomerization can be carried out as a one-pot process. D-Tryptophan is suspended at room temperature in 5 to 50 ml per g of tryptophan, preferably 6 to 12 ml per g of tryptophan of an alcohol, preferably a C₁-C₄-alcohol, more preferably selected from methanol, ethanol or n-propanol; and piperonal is added. The mixture is then treated with an inorganic acid selected from strong inorganic acids, more preferably hydrochloric acid, hydrobromic acid, sulphuric acid, preferably hydrochloric acid, either as concentrated aqueous solutions or as gaseous hydrogen chloride or hydrogen bromide. The amount of acid used is between 1 and 5 equivalents, preferably between 1.1 and 2 equivalents, more preferably 1.1 to 1.9 equivalents, even more preferably between 1.1 and 1.3 equivalents. The solution is heated to about 40 °C to reflux temperature, preferably to 60 to 70 °C and stirred at this temperature for 12 hours to 6 days, preferably for 3 to 4 days. The mixture is then diluted with 5 to 50 ml per g of tryptophan, preferably with 10 to 20 ml per g of tryptophan of water, and the organic solvent is evaporated from the mixture under the reduced pressure. 0.2 to 10 equivalents, preferably 0.5 to 2 equivalents of hydrochloric acid are added, either as an aqueous solution or as hydrogen chloride and the mixture is stirred at a temperature from 0 °C to refluxing temperature, preferably at 50 to 60 °C for 24 to 72 hours.

The above described simplified process represents a direct transformation of D-tryptophan to cis-β-carboline (**8a**) with a much reduced number of technical operations. After finishing the conversion, the mixture is cooled, the precipitate is simply filtered off, optionally dried and further transformed to the chloroacetyl derivative (**5**) by the reaction with chloroacetyl chloride in dichloromethane in the presence of triethylamine or other suitable base. This derivative is then reacted with methylamine in an alcohol in the presence of suitable base to tadalafil.

All Pictet-Spengler reactions according to this invention can be started from L-tryptophan to yield corresponding 1S,3S-cis-β-carboline compounds which are further converted to an enantiomer of tadalafil.

Tadalafil prepared by any of the processes according to the present invention and excipients may be formulated into pharmaceutical formulations according to methods known in the art. Tadalafil produced by the processes of the present invention is suitable for pharmaceutical use in any pharmaceutical formulation.

Tadalafil produced by the processes of the present invention and formulated accordingly can be then used for the treatment of erectile dysfunction or related disorders.

According to the present invention there is provided a method of treating erectile dysfunction or related disorders which comprises administering a therapeutically effective amount of tadalafil in conjunction with a pharmaceutically acceptable diluent or carrier.

The following examples illustrate the process of the present invention and are not intended to limit the scope of the invention:

### Example 1

### Preparation of (1R,3R)-1,2,3,4-tetrahydro-1-(3,4-methylenedioxyphenyl)-9H-pyrido [3,4-b]indole-3-carboxylic acid hydrochloride

D-Tryptophan (7.96 g, 39 mmol) was suspended in THF (60 ml) and the flask was purged with nitrogen. Piperonal (6.46 g, 43 mmol) was added and the mixture was stirred at room temperature for 5 minutes. Concentrated aqueous hydrochloric acid (4 ml) was added and the solution was heated to reflux. The resulting slurry was refluxed for 3 days. The suspension was then cooled to 0 - 5 °C and the product was filtered off, washed with cold THF and dried for 18 hours in vacuum at room temperature to give 9.6 g of the product (98 % area).

### Example 2

### Preparation of (1R,3R)-1,2,3,4-tetrahydro-1-(3,4-methylenedioxyphenyl)-9H-pyrido [3,4-b]indole-3-carboxylic acid hydrochloride

D-Tryptophan (2 g, 10 mmol) was suspended in acetonitrile (150 ml) and the flask was purged with nitrogen. Piperonal (1.65 g, 11 mmol) was added and the mixture was stirred at room temperature for 5 minutes. Concentrated aqueous hydrochloric acid (1 ml) was added and the mixture was heated to reflux. The resulting slurry was refluxed for 3 days. The suspension was then cooled to 0 - 5 °C and the product was filtered off, washed with cold water and dried for 18 hours in vacuum at 40 °C to give 3.1 g of the product (99.8 % area).

### Example 3

### Preparation of (1 R,3R)-1,2,3,4-tetrahydro-1-(3,4-methylenedioxyphenyl)-9H-pyrido [3,4-b]indole-3-carboxylic acid hydrochloride

D-Tryptophan (4 g, 19.6 mmol) was suspended in 1,4-dioxane (40 ml) and the flask was purged with nitrogen. Piperonal (3.25 g, 21.7 mmol) was added and the mixture was stirred at room temperature for 5 minutes. Concentrated aqueous hydrochloric acid (2 ml) was added and the solution was heated to 65 °C. The resulting slurry was stirred at 65 °C for 16 hours. The suspension was then cooled to 0 - 5 °C and the product was filtered off, washed with 1,4-dioxane and dried for 18 hours in vacuum at 40 °C to give 3.6 g of the product (99.7 % area).

### Example 4

### Preparation of methyl (1R,3R)-1,2,3,4-tetrahydro-1-(3,4-methylenedioxyphenyl)-9H-pyrido[3,4-b]indole-3-carboxylate hydrochloride

(1 R,3R)-1,2,3,4-Tetrahydro-1-(3,4-methylenedioxyphenyl)-9H-pyrido[3,4-b]indole-3-carboxylic acid hydrochloride (1 g) was dissolved in methanol (50 ml) and the concentrated hydrochloric acid (1 ml) was added. The mixture was refluxed for 24 hours. The solvent was then evaporated to give approximately 1:1 mixture of cis and trans isomers. 1 M hydrochloric acid (40 ml) was added and the mixture was heated and stirred at 50 °C for 36 hours. The precipitate was filtered off, washed with cold water and dried in vacuum at 40 °C to give 0.63 g of the product.

### Example 5

### Preparation of methyl (1R,3R)-1,2,3,4-tetrahydro-1-(3,4-methylenedioxyphenyl)-9H-pyrido[3,4-b]indole-3-carboxylate hydrochloride

D-Tryptophan (7.96 g, 39 mmol) was suspended in methanol (60 ml) and the flask was purged with nitrogen. Piperonal (6.46 g, 43 mmol) was added and the mixture was stirred at room temperature for 5 minutes. Concentrated hydrochloric acid (4 ml) was added and the mixture was heated to reflux. The solution was refluxed for 4 days. The reaction mixture was evaporated under reduced pressure to approximately 50 % of the original volume and then diluted with dichloromethane (200 ml) and 1 M aqueous NaOH (200 ml). The phases were separated and the aqueous phase was again extracted with dichloromethane (100 ml). Organic phases were combined, washed with brine (100 ml) and evaporated to give approximately 1:1 mixture of cis and trans isomers.
This residue was then heated in a mixture of 1 M hydrochloric acid (50 ml) and water (100 ml) and stirred at 60 °C for 36 hours. The suspension was then cooled to 0 - 5 °C, filtered off, washed with diisopropyl ether and dried in vacuum for 18 hours at 40 °C to give 10.6 g of the product (96 % area).

### Example 6

### Preparation of ethyl (1R,3R)-1,2,3,4-tetrahydro-1-(3,4-methylenedioxyphenyl)-9H-pyrido[3,4-b]indole-3-carboxylate hydrochloride

D-Tryptophan (4 g, 19.6 mmol) was suspended in ethanol (60 ml) and the flask was purged with nitrogen. Piperonal (3.25 g, 21.7 mmol) was added and the mixture was stirred at room temperature for 5 minutes. Concentrated hydrochloric acid (2 ml) was added and the mixture was heated to reflux. The solution was refluxed for 3 days. The reaction mixture was evaporated to approximately 50 % original volume and then diluted with dichloromethane (100 ml) and 1 M aqueous NaOH (100 ml). The phases were separated and the aqueous phase was again extracted with dichloromethane (50 ml). Organic phases were combined, washed with brine (30 ml) and evaporated to give of approximately 1:1 mixture of cis and trans isomers.

This residue was then heated in a mixture of 1 M hydrochloric acid (25 ml) and water (50 ml) at 60 °C for 36 hours. The suspension was then cooled to 0 - 5 °C, filtered off, washed with diisopropyl ether and dried in vacuum for 18 hours at 40 °C to give 4.8 g of the product (96.4 % area).

### Example 7

### Preparation of methyl (1R,3R)-1,2,3,4-tetrahydro-1-(3,4-methylenedioxyphenyl)-9H-pyrido[3,4-b]indole-3-carboxylate hydrochloride

D-Tryptophan (7.96 g, 39 mmol) was suspended in methanol (60 ml) and the flask was purged with nitrogen. Piperonal (6.46 g, 43 mmol) was added and the mixture was stirred at room temperature for 5 minutes. Concentrated sulphuric acid (1.33 ml) was added and the mixture was heated to reflux. The solution was refluxed for 4 days. The reaction mixture was evaporated to approximately 50 % of the original volume and then diluted with dichloromethane (200 ml) and 1 M aqueous NaOH. The phases were separated and the aqueous phase was again extracted with dichloromethane (100 ml). Organic phases were combined, washed with brine (100 ml) and evaporated to give of approximately 1:1 mixture of cis and trans isomers.
This residue was then heated in a mixture of 1 M hydrochloric acid (50 ml) and water (100 ml) and stirred at 60 °C for 36 hours. The suspension was then cooled to 0 - 5 °C, filtered off, washed with diisopropyl ether and dried in vacuum for 18 hours at 40 °C to give 9.8 g of the product (97 % area).

### Example 8

### Preparation of methyl (1R,3R)-1,2,3,4-tetrahydro-1-(3,4-methylenedioxyphenyl)-9H-pyrido[3,4-b]indole-3-carboxylate hydrochloride

D-Tryptophan (4 g, 19.6 mmol) was suspended in methanol (50 ml) and the flask was purged with nitrogen. Piperonal (3.25 g, 21.7 mmol) was added and the mixture was stirred at room temperature for 5 minutes. Concentrated hydrochloric acid (2 ml) was added and the mixture was heated to reflux. The solution was refluxed for 3 days. The reaction mixture was evaporated under reduced pressure to approximately 50 % of the original volume and then diluted with water (50 ml) and the rest of methanol was evaporated from the mixture under reduced pressure. 1 M hydrochloric acid (25 ml) was added and the mixture was stirred at 60 °C for 36 hours. The suspension was then cooled to 0 - 5 °C, filtered off, washed with diisopropyl ether and dried in vacuum for 18 hours at 40 °C to give 4.6 g of the product.

### Example 9

### Preparation of ethyl (1R,3R)-1,2,3,4-tetrahydro-1-(3,4-methylenedioxyphenyl)-9H-pyrido[3,4-b]indole-3-carboxylate hydrochloride

(1R,3R)-1,2,3,4-Tetrahydro-1-(3,4-methylenedioxyphenyl)-9H-pyrido[3,4-b]indole-3-carboxylic acid hydrochloride (1 g) was dissolved in ethanol (50 ml) and the concentrated hydrochloric acid (1 ml) was added. The mixture was refluxed for 24 hours. The solvent was then evaporated to give approximately 1:1 mixture of cis and trans isomers. 1 M hydrochloric acid (40 ml) was added and the mixture was heated and stirred at 50 °C for 36 hours. The precipitate was filtered off, washed with cold water and dried in vacuum at 40 °C to give 0.68 g of the product.

### Example 10

### Preparation of methyl (6R,12aR)-1,2,3,4-tetrahydro-2-chloroacetyl-1-(3,4-methylene dioxyphenyl)-9H-pyrido[3,4-b]indole-3-carboxylate

Methyl (1*R*,3*R*)-1,2,3,4-tetrahydro-1-(3,4-methylenedioxyphenyl)-9H-pyrido[3,4-b]indole-3-carboxylate hydrochloride (3.5 g), prepared by Example 8 was suspended in anhydrous dichloromethane (50 ml) and triethylamine (3.3 ml) was added. The solution was cooled to 0 °C and chloroacetyl chloride (0.91 ml) was added dropwise. The solution was stirred at 0 °C for 6 hours. Isopropanol (30 ml) was added and dichloromethane was evaporated under reduced pressure. The mixture was then cooled and stirred at 0 °C for 2 hours. The solids were filtered off and dried to give 3 g of the product.

### Example 11

### Preparation of (6R,12aR)-2,3,6,7,12,12a-hexahydro-2-methyl-6-(3,4-methylenedioxy phenyl)-pyrazino[2',1':6,1]pyrido[3,4-b]indole-1,4-dione)

Methyl (6R,12aR)-1,2,3,4-tetrahydro-2-chloroacetyl-1-(3,4-methylenedioxyphenyl)-9H-pyrido[3,4-b]indole-3-carboxylate (2.5 g), prepared by Example 10 was suspended in methanol (37.5 ml) at room temperature. A solution of methylamine (40 % in water) (1.5 ml) was added and the flask was purged with nitrogen. The mixture was heated to 50 °C and stirred at 50 °C for 6 hours. The mixture was cooled to 0 °C and stirred at 0 °C for 1 hour. The product was filtered off, washed with methanol and dried to give 2.1 g of product.

### Example 12

### Preparation of (1S,3S)-1,2,3,4-tetrahydro-1-(3,4-methylenedioxyphenyl)-9H-pyrido [3,4-b]indole-3-carboxylic acid hydrochloride

L-Tryptophan (7.96 g, 39 mmol) was suspended in THF (60 ml) and the flask was purged with nitrogen. Piperonal (6.46 g, 43 mmol) was added and the mixture was stirred at room temperature for 5 minutes. Concentrated aqueous hydrochloric acid (4 ml) was added and the solution was heated to reflux. The resulting slurry was refluxed for 3 days. The suspension was then cooled to 0 - 5 °C and the product was filtered off, washed with cold THF and dried for 18 hours in vacuum at room temperature to give 9.3 g of the product (98 % area).

### Example 13

### Preparation of methyl (1S,3S)-1,2,3,4-tetrahydro-1-(3,4-methylenedioxylphenyl)-9H-pyrido[3,4-b]indole-3-carboxylate hydrochloride

L-Tryptophan (32 g, 157 mmol) was suspended in methanol (240 ml) and the flask was purged with nitrogen. Piperonal (26 g, 173 mmol) was added and the mixture was stirred at room temperature for 5 minutes. Concentrated hydrochloric acid (16 ml) was added and the mixture was heated to reflux. The solution was refluxed for 3 days. The reaction mixture was cooled to room temperature and then diluted with ethyl acetate (800 ml) and 1 M aqueous NaOH (800 ml). The phases were separated and the aqueous phase was again extracted with ethyl acetate (400 ml). Organic phases were combined, washed with water (400 ml) and evaporated to give approximately 1:1 mixture of cis and trans isomers.
This residue was then heated in a mixture of 1 M hydrochloric acid (200 ml) and water (400 ml) to 60 °C and stirred at this temperature for 18 hours. The suspension was then cooled to 0 - 5 °C, filtered off, washed with diisopropyl ether and dried in vacuum for 18 hours at 40 °C to give 38 g of the product (93 % area).

## Claims

1. A process for preparing isomerically pure cis-β-carboline compounds of formula (**8a**) and (**S,S-8a**) 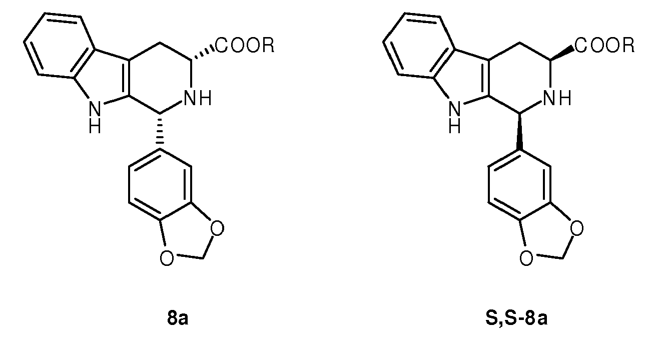 wherein R = H or C₁-C₄-alkyl
comprising the reaction of the suitable isomeric tryptophan (**6, L-6**) with piperonal (**3**) 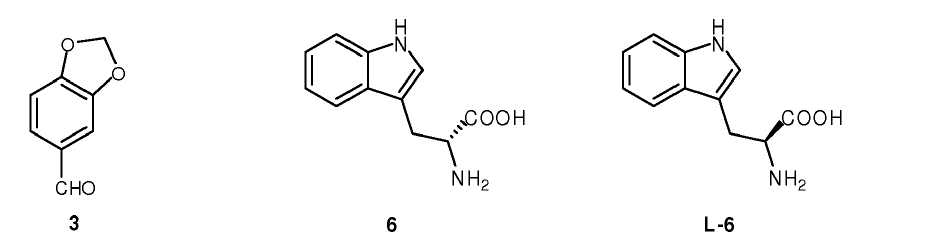 in a solvent in the presence of an inorganic acid whereby said reaction is carried out in one pot.

2. The process according to claim 1 wherein the cis-β-carboline compound of formula (**8a**) is prepared by the reaction of D-tryptophan (**6**) with piperonal (**3**).

3. The process according to claim 1 or 2 wherein said inorganic acid is selected from the group of hydrochloric, hydrobromic and sulphuric acid.

4. The process according to any of claims 1 to 3 wherein R = H and said reaction takes place in a non-chlorinated aprotic solvent in one step without a separation of isomers or isomerization.

5. The process according to claim 4 wherein said solvent is selected from the group of 1,4-dioxane, tetrahydrofuran, 2-methyl-tetrahydrofuran and acetonitrile.

6. The process according to claim 2 wherein R = C₁-C₄-alkyl and said reaction takes place in a C₁-C₄-alcohol without a preparation and isolation of a tryptophan ester.

7. The process according to claim 6 wherein said alcohol is selected from the group of methanol and ethanol.

8. The process according to any of claims 2 to 7 wherein said cis-β-carboline compound is further reacted with chloroacetyl chloride and methylamine to yield tadalafil.

9. The process according to any of claims 2 to 8 wherein said inorganic acid is present in the concentration of 1.1 to 2 equivalents related to tryptophan.

10. The process according to claim 9 wherein said inorganic acid is present in the concentration of 1.1 to 1.3 equivalents related to tryptophan.

11. Use of an inorganic acid together with a non-chlorinated aprotic solvent in the coupling of tryptophan and piperonal.

12. The use according to claim 11 wherein said inorganic acid is present in the concentration of 1.1 to 2 equivalents related to tryptophan.

13. The use according to claims 11 or 12 wherein said tryptophan is D-tryptophan.
